## Europäisches Patentamt
## European Patent Office
## Office européen des brevets

(11) Publication number: **0 108 588**
**B1**

## EUROPEAN PATENT SPECIFICATION

(45) Date of publication of patent specification: **28.06.89**

(51) Int. Cl.⁴: **A 01 N 1/02** // A61K35/14

(21) Application number: **83306631.9**

(22) Date of filing: **31.10.83**

(54) Plasma-free medium for platelet storage and its production.

(30) Priority: **01.11.82 CA 414583**

(43) Date of publication of application:
**16.05.84 Bulletin 84/20**

(45) Publication of the grant of the patent:
**28.06.89 Bulletin 89/26**

(84) Designated Contracting States:
**AT BE CH DE FR GB IT LI NL SE**

(56) References cited:
GB-A-1 014 712
US-A-3 629 071
US-A-3 729 947

CHEMICAL ABSTRACTS, vol. 94, no. 7, 16th
February 1981, page 367, no. 44872f,
Columbus, Ohio, US; E.P.SCOTT et al.:
"Viability and function of platelet concentrates
stored in CPD-adenine(CPDA-1)" &
TRANSFUSION (PHILADELPHIA) 1980, 20(5),
489-97

(73) Proprietor: **Rock, Gail Ann**
270 Sandridge Road
Rockcliffe Park Ottawa Ontario K1L 5A2 (CA)

(72) Inventor: **Rock, Gail Ann**
270 Sandrige Road
Rockcliffe Park, Ontario, K1L 5A2 (CA)
Inventor: **Adams, George Allen**
543 Broadview Avenue
Ottawa, Ontario, K2A 2L3 (CA)

(74) Representative: **Lawrence, Peter Robin
Broughton et al**
GILL JENNINGS & EVERY 53-64 Chancery Lane
London WC2A 1HN (GB)

(56) References cited:
CHEMICAL ABSTRACTS, vol. 77, no. 9, 28th
August 1972, page 308, no. 59601q, Columbus,
Ohio, US; B.L.TRANUM et al.: "In vivo survival
of platelets prepared in CPD citrate-phosphate-
dextroser anticoagulant" & TRANSFUSION
(PHILADELPHIA) 1972, 12(3), 168-74

Courier Press, Leamington Spa, England.

# EP 0 108 588 B1

## Description

This invention is concerned with a method for the storage of blood platelets in a plasma-free medium. Also described is a platelet concentrate obtained by this method.

Thrombocytopenia means low platelet counts in the circulatory system. Persons with this diesease have a tendency to bleed as do hemophiliacs, except that the bleeding is usually from many small capilaries rather than from large vessels as in hemophilia. Such persons suffer small punctate haemorrhages throughout all the body tissues. The skin of such a person will exhibit many small, purplish blotches. Platelets are especially important for repair of minute breaks in capillaries and other small vessels. Platelets aglutinate to fill such ruptures without actually causing clots.

Normally, excessive bleeding does not occur until the number of platelets in the blood falls below a value of approximately 70,000 per cubic millimetre rather than the normal of 150,000 to 350,000.

At the present time, persons who have low platelet counts are supported by infusion of platelet concentrates. Platelet concentrates contain on average $6 \times 10^{10}$ platelets suspended in a volume of 50—60 ml of plasma. Typically they can be made by centrifuging blood plasma to obtain what is commonly termed a pellet but can be described as a consolidated, agglomerated or agglutinated mass having interstitial plasma. Typically a concentrate can be made by dispersing such a pellet in a small volume of plasma. Larger platelet concentrates can be obtained using apheresis machinery in which case the concentration is in the range of $4 \times 10^{11}$ and the plasma volume may reach 200—300 ml. Platelet concentrates can be kept for 3, 5 or even 7 days, depending on the type of bag and mode of rotation used.

There are generally speaking two types of blood collection and storage bags available. One type permits storage of platelets in plasma for up to 3 days while another type permits storage of platelets in plasma for up to 5 days and sometimes up to 7 days. The latter type is generally used for research purposes. In addition it should be noted that platelet storage bags must not contain the plasticizer di-2-ethyl-hexylphthalate and they should be highly permeable. While stored, all bags are subjected to rotation on a continuous basis which can be rotational about a transverse axis or horizontal and reciprocal or horizontal and circular.

The possibility of obtaining a platelet concentrate and storing it in some manner other than those discussed above has been examined in literature but no successful methods have been developed. For instance, it is reported at Chemical Abstracts Vol. 77 1972 page 314 No. 73201R that the storing of platelets as packed cells or in saline resulted in rapid reduction of sensitivity and that to preserve aggregability platelets should be kept in plasma at pH 7.2 to 7.4. In US Patent Specification No. 2 786 014 a process is described in which platelets are centrifuged, eluted using a saline solution and then preserved as a gel in gelatine. The resultant product is thus different from fluid concentrates of the type that are desired and achieved in the present invention.

According to the invention there is provided a new storage-stable substantially plasma-free platelet concentrate comprising blood platelets in a balanced salt solution for use for administration to a human or animal.

The invention also comprises a method of storing blood platelets in stable form in which a platelet concentrate comprising blood platelets in a balanced salt solution and being substantially free of plasma is stored.

Preferably the concentrate contains an anti-coagulant, more preferably selected from acid-citrate-dextrose, citrate-phosphate-dextrose and heparin.

The solution is suitable for administration to animals and preferably is a fluid composition. Preferably it is not a gel so should not contain gelatine or other gel forming component.

The concentrate may be made by a new method comprising centrifuging blood plasma to obtain a platelet pellet, removing substantially all the supernatant plasma and resuspending the platelet pellet in a balanced salt solution that is substantially free of plasma.

By the invention it is possible to store platelets in a medium other than plasma while still retaining the functional characteristics of platelets stored in plasma. The concentrates obtained by the invention typically have a concentration of at least $10^8$, and generally at least $10^9$, platelets per ml of concentrate.

The plasma can be derived from freshly collected whole blood or may be collected using apheresis machinery, i.e. apheresis platelets. The blood or plasma is preferably collected from humans.

Conventional saline solution generally is not satisfactory in the invention and instead a balanced salt solution or salt medium should be used. Many conventional balanced salt solutions can be used in the invention and include Spinner salt solution (Eagle H, Science, 130:432 (1959)), Tyrode's (Tyrode, M. V., Arch. Intern. Pharmacodyn., 20:205 (1910)), Seligmann balanced salt solution (formula below), Earle's balanced salt solutions (Natl. Cancer. Inst. 4:167 (1943)), Dulbecco's phosphate buffered saline (J. Exp. Med., 98:167 (1954)), Hank's balanced salt solutions (Proc. Soc. Exp. Biol. Med., 71:196 (1949)), modification-National Institutes of Health, Gey's balanced salt solutions (Amer. J. of Cancer, 27:55 (1936)), Puck's saline (Puck, T. T., Cieciura, S. J. and Robinson, A. J. Exp. Med., 108:945 (1958)).

The formula for Seligmann balanced salt solution is as follows:

| Component | mg/L |
|---|---|
| NaCl | 7650.00 |
| KCl | 200.00 |
| $NaCO_2 . CH_3$ | 1500.00 |
| $NaH_2PO_4 . H_2O$ | 50.00 |
| $KH_2PO_4$ | 100.00 |
| D-Glucose | 1000.00 |
| $NaHCO_3$ | 700.00 |
| Ascorbic Acid | 3.00 |

The salt solution is preferably an isotonic solution and will contain a number of elements, such as sodium, chlorine, potassium, magnesium and calcium in proportions that produce minimal trauma for the platelets. Typical balanced salt solutions comprise a blend of sodium chloride, potassium chloride, sodium bicarbonate, calcium chloride and magnesium chloride and some are listed in Table 1 below.

The balanced salt solution preferably is formed by adding to a conventional physiologically tolerable isotonic solution one or more additives that enhance platelet stability.

The agents which can be added to the storage medium can include one or more of the following. Nutrients may be added to the medium, and may be selected from fructose and other sugars, adenine or acetyl CoA. These nutrients are substrates for the glycolytic or proteolytic enzymes on the platelet surface and prevent these enzymes from altering the platelet surface.

Another approach is to inhibit the above and other enzymes with reversible inhibitors that are diluted upon infusion into the circulatory system and hence no longer inhibitory. Examples of these compounds are indomethacin, quinacrine or vitamin E, all of which inhibit platelet activation during storage and perhaps increase storage life.

Yet another method to control platelet activation during storage is to raise cyclic adenosine monophosphate with exogenous prostaglandins $E_1$, $D_2$ or $I_2$ which again have a short half-life *in vivo* and reversible effects on platelets.

Finally, the artificial medium can be buffered by addition to the medium of a number of agents all of which can safely be infused into patients. These include phosphate and the amino acids: histidine, cysteine, tyrosine, lysine or arginine. These amino acids have the ability to buffer at an alkaline pH of 9 while phosphate precipitates at this pH.

Thus, in summary the isotonic solution may contain nutrients to improve the storage life of the platelets, reversible inhibitors for platelet activation, substances to raise cyclic adenosine monophosphate levels and which have reversible effects on platelets, and buffering agents which can be safely infused into patients. Generally these additives are used in physiological salt concentrations.

The balanced salt medium preferably contains an anticoagulant, the choice of which is dependent on how the platelets are to be used. The preferred anticoagulants are ACD (acid-citrate-dextrose) and CPD (citrate-phosphate-dextrose). Heparin could be used. Other anticoagulants could be used but they are generalized serine protease inhibitors and would be toxic to human patients. The preferred quantities for ACD and CPD are 1:7 to 1:25 anticoagulant to blood ratio (or 14 mM to 50 mM citrate). Heparin is preferably used in the range of from 3 units/ml to 20 units/ml. Instead of using heparin EDTA could be used in the range of from 2 mM up to 10 mM, but this is generally less preferred since EDTA may have undesirable permanent effects on the platelets.

As a result of the invention the storage of platelet concentrates in a plasma free medium can be effected for at least 72 hours and results can be obtained which are similar to or better than those currently found using standard conditions of storage in plasma. The present platelet concentrates can be stored for the same amount of time as presently available plasma concentrates can be stored. Further the platelet concentrations in the non-plasma medium are about the same as for the plasma medium, that is, about $10^9$/ml or $10^{12}$/L.

There are several advantages to using a balanced salt solution in place of plasma and these are as follows. The plasma can be recovered and can be used for other purposes involving protein fractionation or transfusion to patients. Plasma is an expensive commodity and generally in short supply. Replacement of the plasma as a supernatant for platelet storage would result in considerable saving of plasma (estimated 14,000 litres in Canada alone in 1982) throughout the world.

Another advantage is the possibility of enhancing the storage environment above that generally found for plasma. Platelets are collected into an anticoagulant solution whose pH and biochemical constituents are chosen to enhance red cell preservation and which therefore may not be of optimal composition for platelet storage.

Besides the economic advantages, there are considerable theoretical medical advantages to removal of plasma and resuspension of platelets in a plasma free medium.

i. Removal of plasma components that are potentially harmful to platelets such as glycolytic and proteolytic enzymes that remove membrane glycoproteins and thus cause premature clearance from the circulation;

ii. Reduce the risk of exposure to infectious agents;

iii. Reduced risk of immediate and delayed allergic responses of recipients;

iv. Greater control over environmental conditions, especially pH, ion concentration and volume;

v. Reduced variations in platelet concentrates that would enhance the confidence of physicians in platelet concentrates perhaps leading to therapeutic regimes requiring fewer platelet concentrates per treatment; and

vi. The addition of agents to the storage medium may further improve platelet function after storage or prolong the storage life of platelets.

The following examples are used to illustrate the present invention. All parts and percentages are by weight unless otherwise specified.

Two series of experiments were carried out to show the effects of storing platelets in a non-plasma medium. The first series (A) uses extraction and a washing step to remove plasma from platelets prior to final suspension in a Tyrode's solution. The second series (B) uses only extraction of plasma and final suspension in a modified-Tyrode's solution containing either extra phosphate buffer or histidine buffer.

Example 1
Series A
Method:

Preparations of Platelet Concentrates: Platelet concentrates were prepared by acidification of a pool of platelet-rich plasma using 35 ml acid-citrate-dextrose anticoagulant to 230 ml of platelet-rich plasma, to yield a final pH of 6.4. The platelets were then centrifuged by the normal centrifugation procedure of 3000 G for 5.5 minutes. The lower pH was desirable to facilitate immediate resuspension of the platelet pellet.

The plasma was extracted and the platelets washed by being resuspended in a washing solution containing 0.5 mM EDTA in calcium and magnesium-free Tyrode's buffer, pH 6.4. After centrifugation at 3000 G for 5.5 minutes and extraction of supernatant, the washed platelets were resuspended in CPD-Tyrodes solution or CPD-plasma at a final volume of 60 ml in PL 146 plastic bags (a trade mark of Fenwell Company, Deerfield, Illinois, United States of America). The composition of the CPD-Tyrode's balanced salt solution is given in Table 1. These concentrates wree stored at 22°C in a horizontal shaker for testing after 3 days.

Platelet Aggregation: Aliquots from the platelet concentrates were diluted in pooled plasma containing CPD to a final concentration of $3 \times 10^8$ platelets/μl. After incubating for one hour at 37° the platelet suspensions were aggregated by addition of one stimulus or simultaneous addition of pairs of stimuli. The stimuli and concentrations used were adenosine diphosphate (ADP) at $10^{-5}$M, epinephrine at $5 \times 10^{-5}$M, collagen at 2.4 μg/ml, arachidonic acid at $10^{-4}$M, and calcium ionophore A23187 at $5 \times 10^{-6}$M. All these are final concentrations in the platelet suspensions.

Results:

The results for the platelets washed and resuspended in CPD-Tyrode's (Table 2), and CPD-plasma (Table 3) and for unwashed normal platelet concentrates (Table 4) are presented. The reported values are means and standard errors of at least three determinations except for those aggregations using arachidonate. Although the platelets stored in CPD-Tyrode's had slightly reduced aggregation responses when compared to the two control preparations stored in CPD-plasma, the values are satisfactory. All three preparations lost the ability to aggregate to collagen, epinephrine or ADP while retaining aggregation response to pairs of stimuli or ionophore. The response of all three preparations were identical when tested before storage, indicating the washing procedure did not acutely alter the platelets (data not shown).

Example 2
Series B
Method:

Preparation of Platelet Concentrates: Platelet pellets were prepared by the normal centrifugation procedure of 3000 G for 5.5 minutes (no ACD was added). The plasma was extracted and the platelets resuspended in CPD-Tyrode's phosphate solution, CPD-Tyrode's-Histidine (see Table 1 for recipes) or CPD-plasma at a final volume of 60 ml in PL 145 bags. These concentrates were stored at 22°C in a horizontal shaker for testing after 3 days.

Platelet Aggregation: All aggregations were performed as in Series A.

Results:

The results for the unwashed platelets resuspended in CPD-Tyrode's-phosphate (Table 5), and CPD-Tyrode's-Histidine (Table 6) and for unwashed normal platelet concentrates (Table 4) are presented and show values equal to or better than for platelets stored in the usual way in plasma. The reported values are means and standard errors of at least three determinations except for those aggregations using arachidonate. There was no difference between the platelets stored in CPD-Tyrode's Histidine, PCD-Tyrode's-phosphate and the preparations stored in CPD-plasma. As in Series A, all three preparations lost the ability to aggregate to collagen, epinephrine or ADP while retaining aggregation response to pairs to stimuli or ionophore. The responses of all three preparations were identical when tested before storage, indicating the washing procedure did not significantly alter the platelets (data not shown).

4

### TABLE 1
#### Composition of some artificial mediums used

| | CPD-Tyrode's | CPD-Tyrode's-phosphate | CPD-Tyrode's-histidine |
|---|---|---|---|
| NaCl | 120 mM | 102 mM | 102 mM |
| KCl | 2.4 mM | 2.4 mM | 2.4 mM |
| NaHCO$_3$ | 22.0 mM | 10.0 mM | 10.0 mM |
| NaH$_2$PO$_4$ | 0.4 mM | 22.0 mM | — |
| CaCl$_2$ | 1.8 mM | 1.8 mM | 1.8 mM |
| MgCl$_2$ | 0.9 mM | 0.9 mM | 0.9 mM |
| Glucose | 22.0 mM | 22.0 mM | 22.0 mM |
| Citrate | 1.0 mM | 1.9 mM | 1.9 mM |
| Trisodium Citrate | 10.8 mM | 10.8 mM | 10.8 mM |
| Na$_2$HPO$_4$ | 1.9 mM | 1.9 mM | — |
| Histidine | — | — | 22.0 mM |
| pH | 7.4 mM | 7.4 mM | 7.4 mM |
| Osmolarity | 298 mOsm | 303 mOsm | 313 mOsm |

### TABLE 2
#### Percent platelet aggregation after storage of washed platelets at 22°C for 72 hours in CPD-Tyrode's

| Second stimulus | First stimulus | | | | |
|---|---|---|---|---|---|
| | Saline | ADP | Epinephrine | Collagen | Arachidonate |
| Saline | 0 | | | | |
| ADP | 5(2)* | | | | |
| Epinephrine | 2(3) | 24(8) | | | |
| Collagen | 1(2) | 26(2) | 22(18) | | |
| Arachidonate | 18(5) | — | 34 | — | |
| Ionophore | 31(8) | 29(8) | 27(7) | 31(9) | — |

*mean (S.E.) of at least 3 determinations except for aggregations using arachidonate

TABLE 3

Percent platelet aggregation after storage of washed platelets at 22°C for 72 hours in CPD-plasma

| Second stimulus | First stimulus | | | | |
|---|---|---|---|---|---|
| | Saline | ADP | Epinephrine | Collagen | Arachidonate |
| Saline | 0 | | | | |
| ADP | 8(5)* | | | | |
| Epinephrine | 1(1) | 40(4) | | | |
| Collagen | 2(2) | 43(3) | 47(6) | | |
| Arachidonate | 21(2) | 48 | 46 | — | |
| Ionophore | 40(2) | 45(6) | 40(4) | 47(10) | 46 |

*mean (S.E.) of at least 3 determinations except for aggregations using arachidonate

TABLE 4

Percent platelet aggregation after storage of unwashed platelets at 22°C for 72 hours in CPD-plasma

| Second stimulus | First stimulus | | | | |
|---|---|---|---|---|---|
| | Saline | ADP | Epinephrine | Collagen | Arachidonate |
| Saline | 0 | | | | |
| ADP | 14(5)* | | | | |
| Epinephrine | 0(1) | 40(2) | | | |
| Collagen | 2(1) | 43(2) | 41(2) | | |
| Arachidonate | 27(3) | 44(2) | 43(5) | — | |
| Ionophore | 47(5) | 46(2) | 39(2) | 46(2) | 44 |

*mean (S.E.) of at least 7 determinations except for aggregations using arachidonate which are means of 3 determinations.

TABLE 5

Percent platelet aggregation after storage of unwashed platelets at
22°C for 72 hours in CPD-Tyrode's-phosphate

| Second stimulus | First stimulus | | | | |
|---|---|---|---|---|---|
| | Saline | ADP | Epinephrine | Collagen | Arachidonate |
| Saline | 0 | | | | |
| ADP | 7 | | | | |
| Epinephrine | 0 | 38 | | | |
| Collagen | 2 | 47 | 41 | | |
| Arachidonate | 28 | 40 | 39 | 38* | |
| Ionophore | 40 | 45 | 42 | 46 | 38* |

6

TABLE 6
Percent platelet aggregation after storage of unwashed platelets at
22°C for 72 hours in CPD-Tyrode's-histidine

| Second stimulus | First stimulus | | | | |
|---|---|---|---|---|---|
| | Saline | ADP | Epineprhine | Collagen | Arachidonate |
| Saline | 0 | | | | |
| ADP | 11 | | | | |
| Epinephrine | 1 | 35 | | | |
| Collagen | 5 | 43 | 33 | | |
| Arachidonate | 24* | 42* | 38* | 33* | |
| Ionophore A23187 | + | 48 | 37 | 43 | 30* |

*mean (S.E.) of at least 3 determinations except for aggregation using arachidonate
+2 experiments gave no aggregation with ionophore A23187 while one gave 36% aggregation.
Normal platelet aggregation to ionophore A23187 were found in CPD-Tyrode's-Phosphate.

**Claims**

1. A storage-stable substantially plasma-free platelet concentrate comprising blood platelets in a balanced salt solution for use for administration to a human or animal.

2. A concentrate according to claim 1 comprising an anticoagulant.

3. A concentrate according to claim 2 in which the anticoagulant in the balanced salt solution is selected from acid-citrate-dextrose, citrate-phosphate-dextrose and heparin.

4. A concentrate according to any preceding claim in which the solution contains one or more additives to enhance the stability of the platelets.

5. A concentrate according to any of claims 1 to 3 in which the solution contains one or more additives selected from nutrients to improve the storage life of platelets, reversible inhibitors for platelet activation, substances to raise cyclic adenosine monophosphate levels and have reversible effects on platelets and buffering agents which can be infused into patients.

6. A concentrate according to claim 5, in which the solution contains one or more additives selected from
(a) nutrients selected from fructose and other sugars, adenine and acetyl CoA,
(b) reversible inhibitors for platelet activation selected from indomethacin, quinacrine or vitamin E,
(c) substances to raise cyclic adenosine monophosphate levels selected from prostaglandins $E_1$, $D_2$ and $I_2$, and
(d) buffering agents selected from phosphate and amino acids, preferably selected from histidine, cysteine, tyrosine, lysine and arginine.

7. A concentrate according to any preceding claim, in which the balanced salt solution is selected from Spinner salt solution, Tyrode's solution, Seligmann balanced salt solution, Earle's balanced salt solutions, Dulbecco's phosphate buffered saline, Hank's balanced salt solutions, Gey's balanced salt solutions and Puck's saline.

8. A concentrate according to any preceding claim in which the balanced salt solution is a Tyrode's solution containing a phosphate buffer or a histidine buffer.

9. A concentrate according to any preceding claim, in which the platelets are human platelets.

10. A method for making a storage stable blood platelet concentrate according to claim 1 comprising centrifuging plasma containing platelets to obtain a platelet pellet and resuspending the pellet characterised in that substantially all the supernatent plasma is removed from the platelet pellet and the pellet is then resuspended in a balanced salt solution which is substantially free of plasma.

11. A method according to claim 10 in which the plasma is human plasma derived from freshly collected whole blood or by apheresis.

12. A method according to claim 10 or 11 in which the balanced salt solution comprises the components as defined in any of claims 2 to 9.

13. A method according to any of claims 10 to 12, in which, after removing the supernatant plasma, the pellets are washed to remove plasma from them prior to final suspension in the balanced salt solution.

EP 0 108 588 B1

14. A method of storing blood platelets in stable form in which a platelet concentrate comprising blood platelets in a balanced salt solution and being substantially free of plasma is stored.

15. A method according to claim 14 in which the concentrate is stored for a period of 72 hours.

16. A method according to claim 10 in which the platelet concentrate is a concentrate according to any of claims 1 to 9 or the product of a process according to any of claims 10 to 13.

**Patentansprüche**

1. Lagerstabiles, im wesentlichen plasmafreies Plättchenkonzentrat, enthaltend Blutplättchen in einer im Gleichgewicht befindlichen Salzlösung zur Verwendung zur Verabreichung an einen Menschen oder ein Tier.

2. Konzentrat nach Anspruch 1, dadurch gekennzeichnet, daß es ein Anti-Koagulens enthält.

3. Konzentrat nach Anspruch 2, dadurch gekennzeichnet, daß das Anti-Koagulans in der im Gleichgewicht befindlichen Salzlösung aus Säure-Citrat-Dextrose, Citrat-Phosphat-Dextrose und Heparin ausgewählt ist.

4. Konzentrat nach einem der vorstehenden Ansprüche, dadurch gekennzeichnet, daß die Lösung ein oder mehrere Additive zur Erhöhung der Stabilität der Plättchen enthält.

5. Konzentrat nach einem der Ansprüche 1 bis 3, dadurch gekennzeichnet, daß die Lösung ein oder mehrere Additive, ausgewählt aus Nährmitteln zur Verbesserung der Lagerungslebensdauer der Plättchen, reversiblen Hemmern für die Plättchenaktivierung, Substanzen zur Erhöhung der Gehalte an cyclischem Adenosin-Monophosphat, die reversible Effekte auf die Plättchen haben, und Puffermittel, die Patienten infundiert werden können, enthält.

6. Konzentrat nach Anspruch 5, dadurch gekennzeichnet, daß die Lösung ein oder mehrere Additive, ausgewählt aus

(a) Nährmitteln, ausgewählt aus Fructose und anderen Zuckern, Adenin und Acetyl-CoA,

(b) reversiblen Hemmern für die Plättchenaktivierung, ausgewählt aus Indomethacin, Chinacrin oder Vitamin E,

(c) Substanzen zur Erhöhung der Gehalte an cyclischem Adenosin-Monophosphat, ausgewählt aus Protaglandinen $E_1$, $D_2$ und $I_2$; und

(d) Puffermitteln, ausgewählt aus Phosphat und Aminosäuren, vorzugsweise ausgewählt aus Histidin, Cystein, Tyrosin, Lysin und Arginin, enthält.

7. Konzentrat nach einem der vorstehenden Ansprüche, dadurch gekennzeichnet, daß die im Gleichgewicht befindliche Salzlösung aus Spinner-Salzlösung, Tyrode-Lösung, im Gleichgewicht befindlicher Seligmann - Salzlösung, im Gleichgewicht befindlichen Earl - Salzlösungen, phosphatgepufferter Dulbecco - Kochsalzlösung, im Gleichgewicht befindlichen Hank - Salzlösungen, im Gleichgewicht befindlichen Gey-Lösungen und Puck-Kochsalzlösung ausgewählt ist.

8. Konzentrat nach einem der vorstehenden Ansprüche, dadurch gekennzeichnet, daß die im Gleichgewicht befindliche Salzlösung eine Tyrode-Lösung ist, die einen Phosphat-Puffer oder einen Histidin-Puffer enthält.

9. Konzentrat nach einem der vorstehenden Ansprüche, dadurch gekennzeichnet, daß die Plättchen Human-Plättchen sind.

10. Verfahren zur Herstellung eines lagerstabilen Blutplättchenkonzentrats nach Anspruch 1, bei dem Plättchen enthaltendes Plasma zentrifugiert wird, um ein Plättchen-Pellet zu erhalten und das Pellet resuspendiert wird, dadurch gekennzeichnet, daß man von dem Plättchen-Pellet im wesentlichen das gesamte überstehende Plasma entfernt und sodann des Pellet in einer im Gleichgewicht befindlichen Salzlösung, die im wesentlichen von Plasma frei ist, resuspendiert.

11. Verfahren nach Anspruch 10, dadurch gekennzeichnet, daß das Plasma von frisch gesammeltem Gesamtblut abgeleitetes oder durch Apherese erhaltenes Human-Plasma ist.

12. Verfahren nach Anspruch 10 oder 11, dadurch gekennzeichnet, daß die im Gleichgewicht befindliche Salzlösung die Komponenten nach einem der Ansprüche 2 bis 9 enthält.

13. Verfahren nach einem der Ansprüche 10 bis 12, dadurch gekennzeichnet, daß man nach Entfernung des überstehenden Plasmas vor der Endsuspendierung in der im Gleichgewicht befindlichen Salzlösung die Pellets wäscht, um daraus Plasma zu entfernen.

14. Verfahren zur Lagerung von Blutplättchen in stabiler Form, dadurch gekennzeichnet, daß man ein Plättchenkonzentrat, das Plutplättchen in einer im Gleichgewicht befindlichen Salzlösung, die im wesentlichen von Plasma frei ist, enthält, lagert.

15. Verfahren nach Anspruch 14, dadurch gekennzeichnet, daß man das Konzentrat über einen Zeitraum von 72 Stunden lagert.

16. Verfahren nach Anspruch 10, dadurch gekennzeichnet, daß das Plättchenkonzentrat ein Konzentrat nach einem der Ansprüche 1 bis 9 oder das Produkt eines Verfahrens nach einem der Ansprüche 10 bis 13 ist.

**Revendications**

1. Concentré plaquettaire pratiquement dépourvu de plasma, stable à l'entreposage, comprenant des

8

plaquettes sanguines dans une solution de sels équilibrée, destiné à être administré à l'homme ou à un animal.

2. Concentré suivant la revendication 1, comprenant un anticoagulant.

3. Concentré suivant la revendication 2, dans lequel l'anticoagulant dans la solution de sels équilibrée est choisi entre un mélange acids-citrate-dextrose, un mélange citrate-phosphate-dextrose et l'héparine.

4. Concentré suivant l'une quelconque des revendications précédentes, dans lequel la solution contient un ou plusieurs additifs destinés à accroître la stabilité des plaquettes.

5. Concentré suivant l'une quelconque des revendications 1 à 3, dans lequel la solution contient un ou plusieurs additifs choisie entre des substances nutritives destinées à améliorer la durée de vie des plaquettes à l'entroposage, des inhibiteurs reversibles d'activation plaquettaire, des substances destinées à accroître les teneurs en adénosine-monophosphate cyclique et ayant des effets réversibles sur les plaquettes et des tampons qui peuvent être administrés à des patients par perfusion.

6. Concentré suivant la revendication 5, dans lequel la solution contient un ou plusieur additifs choisis entre

(a) des substances nutritives choisies entre le fructose et d'autres sucres, l'adénine et l'acétyl-CoA,

(b) des inhibiteurs réversibles d'activation plaquettaire choisie entre l'indométhacine, la quinacrine et la vitamine E,

(c) des substances destinées à accroître les teneurs en adénosine-monophosphate cyclique, choisies entre les prostaglandines $E_1$, $D_2$ et $I_2$, et

(d) des tampons choisis entre un phosphate et des amino-acides, choisis de préférence entre l'histidine, la cystéine, la tyrosine, la lysine et l'arginine.

7. Concentré suivant l'une quelconque des revendications précédentes, dans lequel la solution de sels équilibrée est choisie entre la solution de sels de Spinner, la solution de Tyrode, la solution de sels équilibrée de Seligmann, les solutions de sels équilibrées de Earle, le sérum physiologique tamponné avec un phosphate de Dulbecco, les solutions de sels équilibrées de Hank, les solutions de sels équilibrées de Gey et le sérum physiologique de Puck.

8. Concentré suivant l'une quelconque des revendications précédentes, dans lequel la solution de sels équilibrée est une solution de Tyrode contenant un tampon au phosphate ou un tampon à l'histidine.

9. Concentré suivant l'une quelconque des revendications précédentes, dans lequel les plaquettes sont des plaquettes humaines.

10. Procédé de production d'un concentre plaquettaire stable à l'entreposage suivant la revendication 1, consistant à centrifuger du plasma contenant des plaquettes pour obtenir un culot plaquettaire et à remettre en suspension le culot, caractérisé en ce que pratiquement la totalité du plasma surnageant est éliminée du culot plaquettaire et le culot est ensuite remis en suspension dans une solution de sels équilibrée qui est pratiquement dépourvue de plasma.

11. Procédé suivant la revendication 10, dans lequel le plasma est du plasma humain dérivé de sang total fraîchement recueilli ou bien obtenu par aphérèse.

12. Procédé suivant la revendication 10 ou 11, dans lequel la solution de sels équilibrée comprend les constituants définis suivant l'une quelconque des revendications 2 à 9.

13. Procédé suivant l'une quelconque des revendications 10 à 12, dans lequel, après élimination du plasma surnageant, les culots sont lavés pour an éliminer le plasma avant la mise en suspension finale dans la solution de sels équilibrée.

14. Procédé d'entreposage de plaquettes sanguines sous forme stable dans lequel un concentre plaquettaire comprenant des plaquettes sanguines dans une solution de sels équilibrée, pratiquement dépourvue de plasma, est entreposé.

15. Procédé suivant la revendication 14, dans lequel le concentré est entreposé pendant un temps de 72 heures.

16. Procédé suivant la revendication 10, dans lequel le concentré plaquettaire est un concentré suivant l'une quelconque des revendications 1 à 9 ou bien le produit obtenu par mise en oeuvre d'un procédé suivant l'une quelconque des revendications 10 à 13.